(19) Europäisches Patentamt
European Patent Office
Office européen des brevets

(11) EP 4 165 013 B1

(12) **FASCICULE DE BREVET EUROPEEN**

(45) Date de publication et mention de la délivrance du brevet:
**12.11.2025 Bulletin 2025/46**

(21) Numéro de dépôt: **21727884.5**

(22) Date de dépôt: **25.05.2021**

(51) Classification Internationale des Brevets (IPC):
***C07C 231/02** (2006.01)* ***A61K 8/00** (2006.01)*
***A61K 31/00** (2006.01)* ***A61Q 19/00** (2006.01)*
***C07C 233/47** (2006.01)* ***C07C 233/49** (2006.01)*

(52) Classification Coopérative des Brevets (CPC):
(C-Sets disponibles)
**C07D 207/16; A61K 8/44; A61K 9/0048;**
**A61Q 19/00; C07C 231/02;** A61K 2800/10 (Cont.)

(86) Numéro de dépôt international:
**PCT/EP2021/063893**

(87) Numéro de publication internationale:
**WO 2021/254734 (23.12.2021 Gazette 2021/51)**

(54) **PROCÉDÉ DE PRÉPARATION À HAUTE TEMPÉRATURE D'UN N-ACYLE D'ACIDE AMINÉ PAR AMIDIFICATION DIRECTE D'UN ACIDE GRAS**

VERFAHREN ZUR HERSTELLUNG EINER N-ACYLAMINOSÄURE DURCH DIREKTE AMIDIFIZIERUNG EINER FETTSÄURE BEI HOHER TEMPERATUR

METHOD FOR PREPARING AN N-ACYL AMINO ACID BY DIRECT AMIDIFICATION OF A FATTY ACID AT HIGH TEMPERATURE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.06.2020 FR 2006296**

(43) Date de publication de la demande:
**19.04.2023 Bulletin 2023/16**

(73) Titulaire: **Société d'Exploitation de Produits pour les Industries Chimiques SEPPIC**
**75321 Paris cedex 07 (FR)**

(72) Inventeurs:
- **PUJOL, Eric**
  **75321 PARIS CEDEX 07 (FR)**
- **ILLOUS, Estelle**
  **81100 CASTRES (FR)**
- **GUILBOT, Jerome**
  **81100 CASTRES (FR)**
- **DA COSTA, Georges, Manuel**
  **81100 CASTRES (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**FR-A1- 3 066 194 US-B2- 9 505 706**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
**C07C 231/02, C07C 233/47;**
**C07C 231/02, C07C 233/49**

## Description

La présente invention est relative à un procédé de préparation d'un composé N-acylé d'acides aminés.

Les dérivés N-acylés d'acides aminés ou également appelés LipoAminoAcides (LAA), sont des agents tensioactifs anioniques, qui sont constitués par une tête polaire provenant d'un résidu d'au moins un acide aminé, ou bien d'un résidu d'(oligo)-peptides ou bien de résidus d'hydrolysats partiels ou totaux de protéines, et par une chaîne hydrocarbonée de nature lipophile, provenant de chlorures d'acides ou d'esters méthyliques ou d'acides gras voire même de triglycérides, eux-mêmes issus de la filière oléochimique.

Ces dérivés N-acylés d'acides aminés, d'(oligo)-peptides ou bien d'hydrolysats partiels ou totaux de protéines, sont communément utilisés tout d'abord comme ingrédients apportant des propriétés moussantes et nettoyantes pour la préparation de compositions cosmétiques, comme par exemple des gels douche ou des shampoings, ou bien comme ingrédients apportant des propriétés biologiques pour la préparation de compositions cosmétiques destinées à prévenir ou corriger les effets inesthétiques de la peau ; lesdites propriétés biologiques sont par exemple des propriétés anti-âges, hydratantes, amincissantes, raffermissantes, éclaircissantes, dépigmentantes, pro-pigmentantes.

Le procédé de préparation communément mis en œuvre pour la préparation de tels dérivés N-acylés d'acides aminés, d'(oligo)-peptides ou bien d'hydrolysats partiels ou totaux de protéines est connu de l'homme du métier sous le nom de réaction de Schotten Baumann.

Un tel procédé est par exemple divulgué dans les brevets américains US 2,463,779 et US 6,703,517, dans la publication J. Am. Oil Chem. Soc. - 78 (1956) 172, et dans les demandes internationales publiées sous les numéros WO 92/21318 et WO 94/26694.

Ce procédé d'acylation comprend une étape préalable de salification de l'acide aminé, suivie d'une étape d'acylation du sel d'aminoacide avec un chlorure d'acide, puis de l'acidification du sel N-acylé obtenu.

La première étape (voir schéma (1)) consiste à neutraliser l'acide aminé, solubilisé préalablement dans de l'eau ou dans un mélange d'eau et d'un co-solvant organique, par une base minérale, le plus souvent de la soude ou de la potasse aqueuse. Le groupement carboxyle se retrouve alors sous une forme ionisée, permettant ainsi une meilleure solubilité de l'acide aminé dans l'eau. Le pH de cette solution aqueuse se situe entre 9,0 et 12,0, ce qui permet de s'assurer que la fonction amine de l'acide aminé n'est pas protonée.

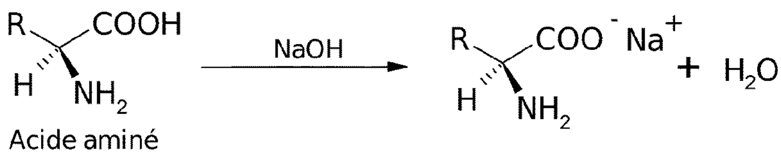


Acide aminé

Schéma 1 : Neutralisation de l'acide aminé.

La seconde étape (voir Schéma 2) est l'étape d'acylation à proprement dite. A ce stade, le chlorure d'acide est ajouté progressivement à la solution neutralisée d'acide aminé, à température ambiante. La fonction amine nucléophile attaque le carbone électrophile de la fonction carbonyle. Il en résulte la formation d'une liaison amide entre les deux substrats de départ ainsi que la formation d'acide chlorhydrique. Cet acide est directement neutralisé in *situ* par ajout progressif d'une base minérale (régulation du pH autour de 10,0).

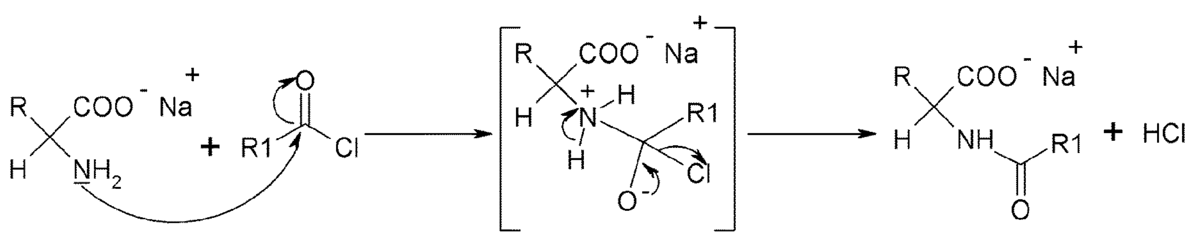

Schéma 2 : Attaque nucléophile de l'acide aminé sur le chlorure d'acide.

A ce stade, la réaction secondaire d'hydrolyse du chlorure d'acide en savon (voir Schéma 3) est également possible. Elle doit cependant être minimisée de façon à atteindre une conversion satisfaisante de l'acide aminé en son dérivé N-acylé et à les isoler efficacement, car une teneur trop élevée en savon peut induire un déphasage du milieu réactionnel et/ou des problèmes d'odeur ou de toxicité (chaines C8 et C11' par exemple).

Les deux principaux paramètres réactionnels qui permettent de contrôler la formation de savon sont :

i) - La vitesse d'agitation lors de la phase réactionnelle, dont l'optimisation permet une amélioration de la surface de contact du chlorure d'acide avec le milieu, et

ii) - l'ajout éventuel, lors de l'étape de solubilisation de l'acide aminé, d'un co-solvant d'acylation, comme par exemple l'acétone, la méthyl éthyl cétone, l'isopropanol, ou des glycols.

Cet ajout de co-solvant permet d'améliorer l'affinité du chlorure d'acide avec le milieu réactionnel. Dans un tel cas, le co-solvant d'acylation doit être choisi judicieusement de façon à éviter ou minimiser la formation de nouveaux produits secondaires issus de la réaction entre ce même co-solvant et le chlorure d'acide, comme par exemple des sous-produits provenant de réactions secondaires d'estérification.

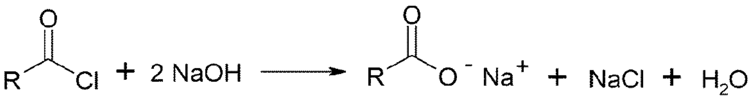

Schéma 3 : Réaction d'hydrolyse du chlorure d'acide (formation de savon).

La dernière étape consiste en une étape de finition, de mise en forme du dérivé N-acylé formé, et trois alternatives sont possibles :

i) La première consiste à régler la valeur du pH du milieu réactionnel obtenu autour de 7. Le dérivé N-acylé est isolé tel quel en solution, sans aucune purification supplémentaire, et comprend les sels d'acylation, les acides aminés non réagis, et l'éventuel co-solvant. Il se présente ainsi sous une forme salifiée, et plus précisément une forme carboxylate, en solution aqueuse et avec une pureté généralement inférieure à 50%.

ii) La seconde consiste à faire précipiter le dérivé N-acylé en acidifiant le mélange réactionnel à une valeur de pH voisine de 2, puis à réaliser plusieurs opérations de filtration et de lavage de la phase organique, se concluant par un séchage final du milieu obtenu.

iii) La troisième, concernant généralement les dérivés N-acylés à bas point de fusion, est basée sur une acidification à chaud (pH ~ 2) de façon à séparer le dérivé N-acylé sous forme acide de la phase aqueuse (appelée "eau mère"). Après soutirage de cette phase aqueuse, plusieurs lavages de la phase organique contenant le dérivé N-acylé peuvent être réalisés par décantation liquide / liquide. Le dérivé N-acylé est finalement mise en forme par figeage et broyage.

Ces deux dernières procédures permettent ainsi d'éliminer tous les sels générés au cours de la réaction d'acylation, l'éventuel co-solvant d'acylation et tous les acides aminés non réagis. Dans ce cas, le dérivé N-acylé d'acide aminé se présente sous une forme non salifiée, avec une fonction acide carboxylique, et une forme solide, plus particulièrement pulvérulente, et avec une pureté supérieure à 80%.

Le procédé Schotten-Baumann précédemment décrit présente comme avantage de conduire des réactions de N-acylation d'acides aminés avec une cinétique rapide, du fait de la réactivité très élevée des chlorures d'acide vis-à-vis des composés et fonctions nucléophiles (par exemple la fonction amine), sans apport important d'énergie comme par exemple l'énergie thermique, dans un milieu solvant majoritairement constitué d'eau, avec des rendements élevés.

Ce procédé comporte en contrepartie comme inconvénients de conduire les réactions en milieux dilués ce qui diminue alors leur productivité ; d'utiliser des chlorures d'acide comme matières premières, obtenus préalablement par des réactions faisant intervenir du chlorure de thionyle ou du trichlorure phosphorique comme réactifs, connus pour leur dangerosité ; d'utiliser des co-solvants organiques difficilement recyclables ; et d'induire d'importantes quantités de sels inorganiques, imposant ainsi le traitement des eaux de lavage.

Pour surmonter ces inconvénients, il existe des alternatives au procédé Schotten-Baumann qui consistent notamment à réaliser une amidification directe d'un acide aminé par un acide gras.

Le document FR3066195A décrit un procédé de préparation de dérivés N-acylés d'acides aminés reposant sur une réaction d'un acide aminé cyclique sur un acide carboxylique gras à une température comprise entre 135°C et 155°C pendant une durée de 4h00 à 10h00. L'ajout d'acide aminé peut être fait par ajout successifs ou par une coulée progressive d'une solution d'acide aminé dilué dans l'eau. Ce procédé concerne des acides aminés cycliques tels que la Proline et l'Hydroxyproline. Leur structure cyclique exacerbe la nucléophilie de la fonction amine endocyclique, ce qui compense la perte de réactivité liée à la substitution des chlorures d'acide par des acides gras.

Le document FR3066194A décrit un procédé de préparation de dérivés N-acylés d'acides aminés consistant à faire réagir à une température d'environ 150°C pendant une durée de 8h00 à 20h00, un acide gras et un acide aminé en présence d'un « pied de réaction ». On appelle « pied de réaction », l'utilisation, comme solvant réactionnel, d'une partie du produit que l'on tente de synthétiser. Dans ce procédé, on utilise donc comme solvant réactionnel le dérivé N-acylé d'acide

aminé que l'on cherche à obtenir. Ce dérivé N-acylé d'acide aminé utilisé comme « pied » peut être obtenu soit via un procédé issu de la demande, soit via un procédé de type Schotten-Baumann. Le dérivé N-acylé d'acide aminé utilisé comme « pied de réaction » est liquide aux températures de réaction envisagées et permet donc de solubiliser l'acide aminé que l'on cherche à amidifier. Le principal inconvénient de cette alternative est la quantité nécessaire de pied de dérivé N-acylé d'acide aminé pouvant constituer jusqu'à 30% massique du milieu réactionnel.

Pour ces deux procédés, les températures et durées de réaction conduisent à des produits finaux très colorés (marrons à noirs), ce qui implique des traitements post-réactionnels obligatoires pour obtenir des produits destinés à être utilisés dans des applications cosmétiques ou pharmaceutiques.

Le document US2016/0052869 décrit une méthode de préparation de dérivés N-acylés d'acides aminés à partir d'acide gras et d'acide aminé à une température comprise entre 115°C et 160 °C, en présence d'un sel déshydratant (comme par exemple le sulfate de magnésium ou le carbonate de magnésium). Le procédé est applicable avec des acides gras comprenant de 2 à 36 carbones, ou avec des acides gras comprenant des cycles, et avec tous les acides aminés.

Partant de là, un problème qui se pose est de fournir un procédé d'amidification amélioré, valable pour toutes les combinaisons d'acides aminés et d'acides gras et caractérisé par un taux de conversion élevé sans utilisation de « pied de réaction » ou de sels déshydratants. Une solution de la présente invention est un procédé de préparation d'un N-acyle d'acide aminé comprenant la réaction d'un acide gras et d'un acide aminé à une température supérieure ou égale à 165°C pendant au moins 1h, de préférence à une température supérieure ou égale à 180°C pendant une durée comprise entre 1 heure et 10 heures.

Le bon déroulement du procédé ne dépend pas de la nature des acides aminés ni de celle des acides gras de départ, ne nécessite pas de « pied de réaction » ni de coulée de solution aqueuse. De surcroît, ce procédé permet de réduire la couleur des produits finaux qui sont de couleurs crème à orangé et non de marron à noir comme dans les procédés antérieurs.

Selon le cas, le procédé selon l'invention peut présenter une ou plusieurs des caractéristiques ci-dessous :

- le N-acyle d'acide aminé est un dérivé N-acylé d'acides aminés de formule (I) :

  R1-C(=O)-Y-OH (I),

  dans laquelle R1-C(=O) représente un radical acyle, linéaire ou ramifié, saturé ou insaturé, comportant de 2 à 36 atomes de carbone et Y représente soit un radical de formule (Ia) :

  - N(R3)-CH(R2)-C(=O) (Ia), dans laquelle R3 représente l'atome d'hydrogène ou le radical méthyle, et R2 représente un atome d'hydrogène ou un radical alkyle, soit un radical de formule (Ib) :

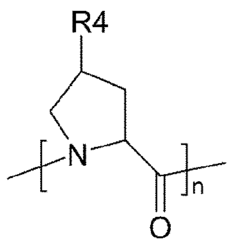

(Ib)

  dans laquelle R4 représente l'atome d'hydrogène ou le radical hydroxyle.

- l'acide gras est de formule (III) :

  R1-C(=O)-OH (III),

  dans laquelle R1-C(=O) représente un radical acyle, linéaire ou ramifié, saturé ou insaturé, comportant de 2 à 36 atomes de carbone
  avec au moins un acide aminé de formule (IIa) :

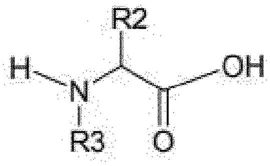

(IIa),

dans laquelle R3 représente l'atome d'hydrogène ou le radical méthyle, et R2 représente un atome d'hydrogène ou un radical alkyle, ou de formule (IIb) :

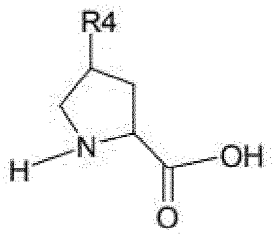

(IIb),

dans laquelle R4 représente un atome d'hydrogène ou un radical hydroxy.

- le radical R2 représente un atome d'hydrogène ou un radical choisi parmi les radicaux méthyle, isopropyle, isobutyle, 1-méthyl propyle, benzyle, 3- amino propyle, hydroxyméthyle, 1-hydroxy éthyle, thiométhyle, (2-méthylthio) éthyle, 4-amino butyle, 3-guanidino propyle, 3-uréido propyle, (1-amino carbonyl) méthyle, carboxy méthyle, 2-carboxy éthyle, 2-(amino carbonyl) éthyle, 4-hydroxy benzyle, 3,4-dihydroxy benzyle, [1H-indol-3-yl] méthyle, (1H-imidazol-4-yl) méthyle.
- Le procédé comprend une étape d'introduction simultanée dans un réacteur de l'acide gras et de l'acide aminé.
- Le procédé comprend : une étape a) dans laquelle l'acide gras est porté à une température supérieure ou égale à 165°C, une étape b) dans laquelle l'acide aminé sous forme solide est versé en une ou plusieurs fois, à une température supérieure ou égale à 150°C, sur l'acide gras issu de l'étape a) pour obtenir un mélange M1, et une étape c) dans laquelle ledit mélange M1 est maintenu sous agitation mécanique pendant au moins une durée d'une heure et à une température supérieure ou égale à 165°C, pour obtenir un mélange aqueux M2 comprenant le N-acyle d'acide aminé.
- le ratio molaire entre l'acide aminé et l'acide gras est compris entre 0,5 et 1,0.
- pendant l'étape c) l'indice d'alcalinité du mélange M1 est mesurée jusqu'à atteindre une valeur inférieure ou égale à 0,2 milli équivalent/gramme de mélange M1.
- Le procédé comprend : une étape a) dans laquelle l'acide gras est porté à une température supérieure ou égale à 165°C, une étape b) dans laquelle l'acide aminé sous forme solide est versé en une ou plusieurs fois, à une température comprise entre 80°C et 120°C, sur l'acide gras issu de l'étape a) pour obtenir un mélange M1, une étape c) dans laquelle ledit mélange M1 est chauffé à une température supérieure ou égale à 165°C, et une étape d) dans laquelle ledit mélange M1 est maintenu sous agitation mécanique pendant au moins une heure et à une température supérieure ou égale à 165°C pour obtenir un mélange aqueux M2 comprenant le N-acyle d'acide aminé.
- pendant l'étape d) l'indice d'alcalinité du mélange M1 est mesuré jusqu'à atteindre une valeur inférieure ou égale à 0,2 milliéquivalent/gramme de mélange M1. Par « indice d'alcalinité », on entend dans le cadre de la présente invention la valeur résultant d'un dosage potentiométrique de la partie anhydre du milieu réactionnel au cours duquel les fonctions amines résiduelles portées par l'acide aminé sont dosées avec un acide fort (acide perchlorique), et en présence d'une électrode adaptée pour permettre de déterminer le potentiel d'équivalence du dosage acide/base et le volume d'acide associé à l'équivalence. La mesure de « l'indice d'alcalinité » est exprimée en milliéquivalent d'acide versé à l'équivalence par gramme d'échantillon dosé, et elle est caractéristique dans le cas présent de l'équivalent molaire de fonction amines non réagies sur l'acide aminé dans le milieu réactionnel. Cette mesure de « l'indice d'alcalinité » est un moyen de suivre l'avancement de la réaction et la conversion des fonctions amines portées par l'acide aminé.
- l'étape a) comprend une première sous-étape de fonte de l'acide gras et une deuxième sous-étape d'homogénéisation de l'acide gras.
- Le procédé comprend une étape supplémentaire d'isolation du N-acyle d'acide aminé du mélange M2.
- Le procédé est réalisé sous atmosphère d'azote.
- L'acide gras est tel que le radical acyle R1-C(=O)- linéaire ou ramifié, saturé ou insaturé, constitutif de l'acide gras de

formule (III) comporte de 6 à 36 carbones, de préférence de 10 à 18 carbones, encore plus préférentiellement de 12 à 18 carbones.

- l'acide aminé est choisi parmi les acides aminés de formule (IIa) dans laquelle R3 représente l'atome d'hydrogène et dans laquelle le radical R2 représente l'atome d'hydrogène ou un radical choisi parmi les radicaux méthyle, isopropyle, isobutyle, 1-méthyl propyle, benzyle, 3-guanidino propyle, [1H-indol-3-yl] méthyle, (1H-imidazol-4-yl) méthyle, 2-carboxy éthyle, ou parmi les acides aminés de formule (IIb) dans laquelle R3 représente l'atome d'hydrogène et dans laquelle le radical R4 représente l'atome d'hydrogène ou le radical hydroxy.

La présente invention a également pour objet un procédé de fabrication d'une composition cosmétique d'une composition cosmétique comprenant la préparation d'un N-acyle d'acide aminé telle que défini précédemment.

Un exemple d'une telle composition cosmétique est une composition cosmétique à usage topique (C) comprenant pour 100% de sa masse :

- de 0,1% à 40% massique, plus particulièrement de 0,1% à 20% massique, et encore plus particulièrement de 0,1% à 5% massique du mélange (M2) telle que défini précédemment, et
- de 60% à 99,9% massique, plus particulièrement de 80% à 99,9%, et encore plus particulièrement de 95% à 99,9% massique d'un milieu cosmétiquement acceptable.

L'expression "à usage topique" utilisée dans la définition de la composition cosmétique à usage topique (C) telle que définie ci-dessus, signifie que ladite composition est mise en œuvre par application sur la peau, les cheveux, le cuir chevelu, les lèvres ou les muqueuses, qu'il s'agisse d'une application directe dans le cas d'une composition cosmétique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

L'expression "cosmétiquement acceptable" utilisée dans la définition de la composition cosmétique à usage topique (C), signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état. Un milieu cosmétiquement acceptable de ces compositions peut contenir classiquement de l'eau, un ou plusieurs solvants organiques cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, l'hexylène glycol, le diéthylène glycol, le xylitol, l'érythritol, le sorbitol, ou les alcools hydrosolubles.

La composition cosmétique à usage topique (C) peut être conditionnée sous forme pressurisée dans un dispositif aérosol ou dans un dispositif de type « flacon pompe », dans un dispositif muni d'une paroi ajourée par exemple une grille, dans un dispositif muni d'un applicateur à billes (dit « roll-on »). Lorsqu'elle est conditionnée dans des flacons, la composition (C) telle que définie précédemment peut être appliquée sous la forme de fines gouttelettes au moyen de dispositifs de pressurisation mécanique ou à gaz propulseur. Parmi les agents propulseurs que l'on peut associer à la composition (C), il y a les composés hydrofluorés, comme le dichlorodifluorométhane, le trichlorofluorométhane, le difluoréthane, l'isobutane, le butane, le propane.

La composition cosmétique à usage topique (C) telle que définie précédemment peut en outre comporter des excipients et/ou des principes actifs habituellement mis en œuvre dans le domaine des formulations à usage topique, en particulier cosmétiques ou pharmaceutiques. La composition cosmétique à usage topique (C) et telle que définie précédemment, peut comprendre en outre, un ou plusieurs composés auxiliaires choisis parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents opacificants, les agents nacrants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Parmi les agents antioxydants que l'on peut associer à la composition cosmétique à usage topique (C), il y a l'acide ascorbique, la glutathione, l'acide tartrique, l'acide oxalique, le tétra sodium glutamate diacétate, la vitamine E et ses dérivés.

Parmi les agents séquestrants hydrosolubles que l'on peut associer à la composition cosmétique à usage topique (C), il y a les sels de l'acide éthylènediamine tétracétique (EDTA), comme le sel de sodium de l'EDTA, les sels de

l'acide diéthylènetriamine pentacétique (DTPA) comme les sels de sodium du DTPA, l'acétyl glutamique acide (gamme Dissolvine).

Parmi les colorants hydrosolubles que l'on peut associer à la composition cosmétique à usage topique (C), il y a le caramel, le Yellow 5, l'Acid Blue 9/ Blue 1, le Green 5, le Green 3 / Fast Green FCF 3, l'Orange 4, le Red 4 / Food Red 1, le Yellow 6, l'Acid Red 33 / Food Red 12, le Red 40, le carmine de cochenille (CI 15850, CI 75470), l'Ext. Violet 2, le Red 6-7, le Ferric Ferrocyanide, les Ultramarines, l'Acide Yellow 3 / Yellow 10, l'Acid Blue 3, le Yellow 10.

Parmi les agents hydrosolubles stabilisant de la couleur que l'on peut associer à la composition cosmétique à usage topique (C), il y a le citrate de Tris(tétraméthyl hydroxy pipéridinol), benzotriazolyl butylphénol sulfonate de sodium, le benzotriazolyl dodecyl p-crésol.

Comme exemples de tensioactifs moussants et/ou détergents, éventuellement présents dans la composition cosmétique à usage topique (C), on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à la composition cosmétique à usage topique (C), on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents éventuellement présents dans la composition cosmétique à usage topique (C), on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans la composition cosmétique à usage topique (C), on peut citer particulièrement les dérivés d'ammoniums quaternaires.

Parmi les tensioactifs non ioniques moussants et/ou détergents éventuellement présents dans la composition cosmétique à usage topique (C), on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines. Comme exemples d'agents de texture éventuellement présents dans la composition cosmétique à usage topique (C) , on peut citer des dérivés N-acylés d'acides aminés, par exemple la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV 14, les fibres de cellulose, les fibres de coton, les fibres de chitosane, le talc, la séricite, le mica, la perlite. Comme exemples de principes actifs éventuellement présents dans la composition cosmétique à usage topique (C), il y a :

- Les vitamines et leurs dérivés, par exemple, le rétinol (vitamine A) et ses esters (palmitate de rétinyle par exemple), l'acide ascorbique (vitamine C) sous forme de sel et ses esters, les dérives de sucre de l'acide ascorbique (par exemple l'ascorbyl glucoside), le tocophérol (vitamine E) et ses esters (par exemple l'acétate de tocophérol), les vitamines B3 ou B10 (niacinamide et ses dérivés) ;
- Les composés ayant une action éclaircissante ou dépigmentante de la peau, par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™ ;
- Les composés ayant une action apaisante comme le SEPICALM™ S, l'allantoïne, l'huile de graines de plantes ombellifères comme par exemple le SEPIBLISS™, et le bisabolol ;
- Les agents anti-inflammatoires comme par exemple le Diclofenac™;
- Les composés montrant une action hydratante par exemple le diglycérol, le triglycérol, l'urée, les hydroxy urées, le glycérol glucoside, le diglycérol glucoside, les polyglycéryl glucosides, l'érythrityl glucoside, le sorbityl glucoside, le xylityl glucoside, la composition commercialisée sous le nom de marque AQUAXYL™ comprenant du xylityl glucoside, de l'anhydroxylitol et du xyllitol ;
- Les composés montrant une action amincissante ou lipolytique comme la caféine ou ses dérivés, l'ADIPOSLIM™, l'ADIPOLESS™ ;
- Les extraits végétaux riches en tanins en polyphénols et/ ou en isoflavones, par exemple les extraits de raisin, les extraits de pin, les extraits de vin, les extraits d'olives ; les extraits de soja, par exemple la Raffermine™ ; les extraits de blé par exemple la TENSINE™ ou la GLIADINE™ ; les extraits végétaux riches en terpènes ; les extraits d'algues d'eau douce ou marines ; les extraits marins en général comme les coraux ;
- Les composés ayant une action antimicrobienne ou une action purifiante, par exemple le LIPACIDE™C8G, le LIPACIDE™UG, le SEPICONTROL™ A5 ; l'OCTOPIROX™ ou le SENSIVA™SC50 ;

- Les composés ayant une propriété énergisante ou stimulante comme le Physiogényl™, le panthénol et ses dérivés comme le SEPICAP™MP ;
- Les actifs anti-âge comme le SEPILIFT™DPHP, le LIPACIDE™PVB, le SEPIVINOL™, le SEPIVITAL™, le MANOLIVA™, le PHYTO-AGE™, le TIMECODE™ ; le SURVICODE™ ;
- Les actifs anti-photovieillissement;
- Les actifs augmentant la synthèse des composants de la matrice extracellulaire par exemple le collagène, les élastines, les glycosaminoglycanes ;
- Les actifs agissant favorablement sur la communication cellulaire chimique comme les cytokines ou physiques comme les intégrines ;
- Les actifs créant une sensation de « chauffe » sur la peau comme les activateurs de la microcirculation cutanée (par exemple les dérivés de l'acide nicotinique) ou des produits créant une sensation de « fraîcheur » sur la peau (par exemple le menthol et des dérivés) ;
- Les actifs améliorant la microcirculation cutanée, par exemple les veinotoniques ; les actifs drainants ; les actifs à visée décongestionnante par exemple les extraits de ginko biloba, de lierre, de marron d'inde, de bambou, de ruscus, de petit houx, de centalla asiatica, de fucus, de romarin, de saule ;
- Les actifs agissant comme agents tenseurs de la peau, par exemple les hydrolysats de protéines végétales, les hydrolysats d'origine marine comme les hydrolysats d'extraits de laminaires, les hydrolysats de cartilages de poissons, l'élastine marine, le produit commercialisé par la société SEPPIC sous le nom de marque SESAFLASH™, les solutions de collagène.
- Les agents de bronzage ou de brunissement de la peau, par exemple la dihydroxyacétone, l'isatine, l'alloxane, la ninhydrine, le glycéraldéhyde, l'aldéhyde mésotartrique, la glutaraldéhyde, l'érythrulose.

Comme exemples d'agents déodorants éventuellement présents dans la composition cosmétique à usage topique (C), on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol, le caprylate de glycérol, le caprate de polyglycérol, le 1,2-décanediol, le 1,3-propanediol, l'acide salicylique, le bicarbonate de sodium, les cyclodextrines, les zéolithes métalliques, le Triclosan™, le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'agents épaississants ou gélifiants éventuellement présents dans la composition cosmétique à usage topique (C) , on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- méthyl)acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymères linéaire, branché ou réticulé d'au moins un monomère possédant une fonction acide fort, libre, partiellement salifiée ou totalement salifiée, avec au moins un monomère neutre, et au moins un monomère de formule (VIII):

$$CH_2=C(R'_3)-C(=O)-[CH_2-CH_2-O]_n-R'_4 \quad (VIII)$$

dans laquelle $R'_3$ représente un atome d'hydrogène ou un radical méthyle, $R'_4$ représente un radical alkyle linéaire ou ramifié comportant de huit à trente atomes de carbone et n représente un nombre supérieur ou égal à un et inférieur ou égal à cinquante;

Les polymères de type polyélectrolytes, linéaires ou branchés ou réticulés que l'on peut associer à la composition cosmétique à usage topique (C), peuvent se présenter sous la forme d'une solution, d'une suspension aqueuse, d'une émulsion eau-dans-huile, d'une émulsion huile-dans-eau, d'une poudre, par exemple les produits commercialisés sous les appellations SIMULGEL™ EG, SIMULGEL™EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSMEDIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100; les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1); les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes; la cellulose, les dérivés de cellulose comme la méthyl-cellulose, l'éthylcellulose, l'hydroxypropyl cellulose, les silicates, l'amidon, les dérivés hydrophiles de l'amidon, les polyuréthanes.

Comme exemples d'huiles éventuellement présentes dans la composition cosmétique à usage topique (C), on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de graines de coriandre, l'huile de faines, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires éventuellement présentes dans la composition cosmétique à usage topique (C), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de tensioactifs non-ioniques émulsionnants que l'on peut associer à la composition cosmétique à usage topique (C), on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre cinq moles et cent cinquante moles d'oxyde d'éthylène, comme la composition comprenant de l'acide stéarique éthoxylé à cent trente-cinq moles d'oxyde d'éthylène et du stéarate de glycérol commercialisée sous l'appellation SIMULSOL™ 165 ; les esters de mannitan ; les esters de

mannitan éthoxylés ; les esters de sucrose ; les esters de méthyl glucoside ; les alkyl polyglycosides comportant un radical aliphatique, linéaire ou ramifié, saturé ou insaturé, et comportant de quatorze à trente-six atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le (2-octyl dodécyl) polyxyloside, le (12-hydroxy stéaryl) polyglucoside ; les compositions d'alcools gras linéaires ou ramifiés, saturés ou insaturés, et comportant de quatorze à trente-six atomes de carbone, et d'alkyl polyglycosides tels que décrits précédemment, par exemple les compositions commercialisées sous les noms de marque MONTANOV™68, MONTANOV™14, MONTANOV™82, MONTANOV™202, MONTANOV™S, MONTANOV™WO18, MONTANOV™L, FLUIDANOV™20X et EASYNOV™.

Comme exemples d'agent de protection contre les rayonnements ultra-violets du soleil éventuellement présents dans la composition cosmétique à usage topique (C), on désigne les pigments, les filtres organiques solaires et les filtres inorganiques solaires.

Comme pigments utilisés comme agent de protection contre les rayonnements ultra-violets du soleil éventuellement présents dans la composition cosmétique à usage topique (C), il y a par exemple le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Comme filtres organiques solaires utilisés comme agent de protection contre les rayonnements ultra-violets du soleil éventuellement présents dans la composition cosmétique à usage topique (C), il y a par exemple:

- Ceux de la famille des dérivés de l'acide benzoïque comme les acides paraaminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-$\alpha$-cyano-$\beta$-phényle, le cinnamate de 2-éthylhexyl-$\alpha$-cyano-$\beta$-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- Ceux de la famille des dérivés de l'acide sulfonique comme l'acide 2-phényl benzimidazole-5-sulfonique et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonypagel) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;
- Ceux de la famille des polysiloxanes comme le malonate de benzylidène siloxane.

Comme filtres inorganiques solaires utilisés comme agent de protection contre les rayonnements ultra-violets du soleil éventuellement présents dans la composition cosmétique à usage topique (C), il y a par exemple: les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Les exemples suivants illustrent l'invention sans toutefois la limiter.

**Comparaison entre le procédé selon l'invention (procédé 1) et les procédés comparatifs, dont un est décrit dans le document** FR3066194 **(procédé 2) et l'autre est une variante du procédé 2 se distinguant par**

**l'absence de pied de réaction (procédé 3).**

- Pour rappel le procédé 2 décrit dans le document FR 3066194 est celui-ci :
  - Chargement du réacteur avec l'acide gras de formule (III), fonte et homogénéisation sous agitation à 80°C (milieu limpide).
  - Ajout de l'acide aminé de formule (IIa) et/ou de formule (IIb) et du "pied de réaction".
  - Maintien de la température à 150°C et courant d'azote en atmosphère.
  - Arrêt et vidange sous atmosphère d'azote.

Le pied de réaction est formé du N-acyle d'acide aminé que l'on veut synthétiser et peut provenir de la mise en œuvre ou d'un procédé Shotten Baumann (impliquant un chlorure d'acide gras) ou d'un procédé issu de l'amidification directe (impliquant un acide gras de formule (III)).

- Le procédé 3 est une variante du procédé 2 qui contient les mêmes étapes à l'exception de l'ajout du « pied de réaction » qui n'est pas introduit dans le milieu réactionnel.

- Description du procédé 1 selon l'invention:
  On introduit la quantité d'acide gras de formule (III), de façon à ce qu'elle représente de 0,5 à 1,0 équivalent molaire, dans un réacteur muni d'une double enveloppe contenant un fluide caloporteur, d'un agitateur mécanique équipé d'une pale d'agitation de type ancre ou hélice, et d'un dispositif de condensation à eau. Pendant l'introduction de l'acide gras, la température est portée à 80°C de façon à fondre l'acide gras si nécessaire.

Par la suite, la température est portée à 150°C, et la quantité de 1,0 équivalent molaire d'acide aminé de formule (IIa) ou de formule (IIb), ou d'un mélange d'acides aminés de formule (IIa) et/ou de formule (IIb) est introduite sur l'acide gras agité sous forme de solution ou de dispersion aqueuse à la température de 150°C. Cette introduction peut être réalisée en une seule fois ou en plusieurs fractions. Le mélange réactionnel agité est ensuite porté à une température de 180°C sous atmosphère d'azote pendant une durée minimale d'une heure. L'avancement de la réaction est contrôlé par la mesure de « l'indice d'alcalinité » du milieu réactionnel à intervalles réguliers et le refroidissement progressif du milieu réactionnel est engagé lorsque la mesure de l'indice d'alcalinité dudit milieu réactionnel est inférieure ou égale à 0,2 milliéquivalent/gramme de milieu réactionnel.

Le calcul du taux de conversion est le rapport de la quantité molaire d'acide gras ayant réagi sur la quantité molaire d'acide gras introduit, le tout exprimé en pourcentage. Le nombre de moles d'acide gras introduites est calculé par rapport aux charges massiques introduites. Le nombre de moles d'acide gras ayant réagi est calculé par rapport aux pourcentages dosés restant dans le produit fini.

$$\text{Taux de conversion} = n_{moles}\ \text{acide gras réagi} / n_{moles}\ \text{acide gras départ} \times 100$$

$n_{moles}$ acide gras départ = masse acide gras introduite/PM acide gras

$n_{moles}$ acide gras réagi = $n_{moles}$ acide gras départ - (masse produit fini x % acide gras dosé / PM acide gras)

% acide gras dosé : Le dosage en acide gras est un dosage quantitatif dosé par rapport à une solution étalon, réalisé par la mise en œuvre d'une méthode de chromatographie en phase gazeuse.

Les pourcentages d'aire des pics correspondant au N-acyle d'acide aminé ont été mesurés par la mise en œuvre d'une méthode qualitative avec un dispositif de chromatographie en phase gazeuse, et ont été normalisés à 100.

Les exemples suivants illustrent l'invention sans toutefois la limiter.

**I- Exemples de préparation de N-palmitoyl isoleucine par le procédé selon l'invention et des procédés comparatifs**

Exemple 1 préparation de N-palmitoyl isoleucine par la mise en œuvre du procédé 1 selon l'invention

On introduit 213,1 grammes d'acide palmitique (ou acide hexadécanoïque) soit 0,6 équivalent molaire dans un réacteur de deux litres muni d'une double enveloppe contenant un fluide caloporteur, d'un agitateur mécanique équipé d'une pale d'agitation de type ancre ou hélice, et d'un dispositif de condensation à eau. Pendant l'introduction de l'acide gras, la température est portée à 80°C de façon à fondre l'acide gras si nécessaire.

Une fois l'acide gras homogénéisé, on porte l'acide gras fondu à une température de 150°C et on ajoute ensuite 181,7 grammes d'isoleucine soit 1 équivalent molaire.

On porte ensuite le mélange à une température de 180°C en suivant la valeur de l'indice d'alcalinité du milieu réactionnel par mesure à intervalles réguliers de l'indice d'alcalinité jusqu'à ce que celle-ci soit inférieure ou égale à 0,2 milliéquivalent/gramme de milieu réactionnel. Cette valeur seuil a été atteinte après une durée de trois heures.

Le milieu réactionnel est refroidi progressivement et le réacteur est ensuite vidangé pour obtenir la composition LAA1.

Exemple 2 préparation de N-palmitoyl isoleucine par la mise en œuvre du procédé 2

On introduit 213,1 grammes d'acide palmitique (ou acide hexadécanoïque) soit 0,6 équivalent molaire dans un réacteur de deux litres muni d'une double enveloppe contenant un fluide caloporteur, d'un agitateur mécanique équipé d'une pale d'agitation de type ancre ou hélice, et d'un dispositif de condensation à eau. Pendant l'introduction de l'acide gras, la température est portée à 80°C de façon à fondre l'acide gras si nécessaire.

Une fois l'acide gras homogénéisé, on porte l'acide gras fondu à une température de 150°C et on ajoute ensuite une dispersion aqueuse comprenant 181,7 grammes soit 1 équivalent molaire d'isoleucine et une quantité de 100,7 grammes de N-palmitoyle isoleucine à pureté de 98%, préalablement obtenu par un procédé de type Schotten Bauman, soit une proportion massique de 25% (« pied de réaction ») de N-palmitoyl isoleucine dans le milieu réactionnel.

On porte ensuite le mélange à une température de 155°C en suivant la valeur de l'indice d'alcalinité du milieu réactionnel par mesure à intervalles réguliers de l'indice d'alcalinité jusqu'à ce que celle -ci soit inférieure ou égale à 0,2 milliéquivalent/gramme de milieu réactionnel. Cette valeur seuil a été atteinte après une durée de 12h30.

Le milieu réactionnel est refroidi progressivement et le réacteur est ensuite vidangé pour obtenir la composition LAA11.

Exemple 3 préparation de N-palmitoyl isoleucine par la mise en œuvre du procédé 3

Le procédé décrit dans l'exemple 2 est reproduit mais sans l'ajout de la quantité de « pied de réaction » et la température de réaction entre les espèces est de 150°C au lieu de 155°C, pendant une durée de 12h30, pour obtenir la composition LAA12.

Exemple 4 préparation de N-palmitoyl isoleucine par la mise en œuvre du procédé 3

Le procédé décrit dans l'exemple 3 est reproduit mais avec une température de réaction entre les espèces de 160°C au lieu de 150°C, pendant une durée de 3h00, pour obtenir la composition LAA13.

Exemple 5 préparation de N-palmitoyl isoleucine par la mise en œuvre du procédé 3

Le procédé décrit dans l'exemple 3 est reproduit mais avec une température de réaction entre les espèces de 160°C au lieu de 150°C, pendant une durée de 4h30, pour obtenir la composition LAA14.

Exemple 6 préparation de N-palmitoyl isoleucine par la mise en œuvre du procédé 3

Le procédé décrit dans l'exemple 3 est reproduit mais avec une température de réaction entre les espèces de 160°C au lieu de 150°C, pendant une durée de 19h30, pour obtenir la composition LAA15. Les conditions opératoires des exemples **1,** 2, 3, 4, 5 et 6, et les caractéristiques analytiques des compositions LAA1, LAA11, LAA12, LAA13, LAA14 et LAA15 correspondantes, sont consignées dans le tableau 1 ci-après.

[Table 1]

| **Référence des compositions** | **LAA11** | **LAA12** | **LAA1** | **LAA13** | **LAA14** | **LAA15** |
|---|---|---|---|---|---|---|
| **PROCEDE** | **2** | **3** | **1 selon l'invention** | **3** | **3** | **3** |
| **ratio molaire Acide palmitique / isoleucine** | 0,6 | 0,6 | 0,6 | 0,6 | 0,6 | 0,6 |
| **Température de réaction** | **155°C** | **150°C** | **180°C** | **160°C** | **160°C** | **160°C** |
| **Durée du maintien en température de réaction** | 12h30 | 18h30 | 3h00 | 3h00 | 4h30 | 19h30 |

(suite)

| Référence des compositions | LAA11 | LAA12 | LAA1 | LAA13 | LAA14 | LAA15 |
|---|---|---|---|---|---|---|
| Pied de réaction | 25% | Sans | Sans | Sans | Sans | Sans |
| Analyses | | | | | | |
| Aspect à 25°C | Solide Brun | Solide jaune | Solide jaune | Solide Crème | Solide Beige | Solide marron |
| Indice d'Alcalinité (milliéquivalent/gramme) | 0,19 | 1,93 | 0,04 | 2,876 | 2,070 | 0,056 |
| Dosage Acide palmitique résiduel (% massique) | **11,1** | **28,5** | **10,5** | **40,2** | **36,6** | **9,6** |
| Taux conversion molaire Acide palmitique (%) | **74** | **49,4** | **82** | **30,2** | **36,5** | **83,3** |
| Isoleucine résiduelle en % aire | / | 34,3 | / | 40,1 | 37,0 | / |
| Di-isoleucine en % aire | / | 0,2 | 3,9 | / | 0,2 | 2,7 |
| Influence de la nature du procédé de préparation de compositions à base de N-Palmitoyl Isoleucine. | | | | | | |

Au regard des taux de conversion d'acide gras, on constate bien ici que l'utilisation du pied à 150°C est nécessaire. En l'absence de celui-ci, la réaction se fait plus rapidement et avec un taux de conversion plus élevé à condition d'augmenter la température à 180°C.

Le procédé 1 selon l'invention permet d'obtenir une composition LAA1 comprenant une faible teneur en acide palmitique résiduel (10,5%), avec un taux élevé de conversion de l'acide palmitique (82%), sans introduire de « pied de réaction » et donc d'améliorer la productivité du procédé en limitant le coût des matières premières.

D'autre part, le procédé 2 impliquant un « pied de réaction » permet d'atteindre une composition LAA11 comprenant une faible teneur en acide palmitique résiduel (10,5%) avec un taux de conversion plus faible (74%) que celui mesuré dans le procédé 1 (82%), avec une durée de réaction plus longue (12h30) que celle du procédé 1 (3h00).

Lorsque le procédé 3 est mis en œuvre pour préparer les compositions LAA13 et LAA14, avec une température de réaction de 160°C, les taux de conversion de l'acide palmitique sont nettement plus faibles (30,2% pour LAA13 et 36,5% pour LAA14 et les teneurs en acide palmitique résiduel nettement plus élevées (40,2% pour LAA13 et 36,6% pour LAA14).

Le procédé 3 permet d'obtenir une composition LAA15 avec un fort taux de conversion (83,3%) et une faible teneur en acide palmitique résiduel (9,6%) lorsque la durée du maintien à 160°C est de 19h30, ce qui a pour inconvénient de diminuer la productivité et d'obtenir une composition de couleur marron impropre à l'utilisation en formulation à destination pour les industries cosmétiques et pharmaceutiques.

II- **Exemples de préparation de N-Undécylènoyl Phénylalanine par le procédé selon l'invention et par le procédé 2 comparatif**

Exemple 7 préparation de N-undécylènoyl PhénylAlanine par la mise en œuvre du procédé 1 selon l'invention

On reproduit l'exemple 1 en remplaçant les 213,1 grammes d'acide palmitique par 109,7 grammes d'acide undécylénoyle, les 181,7 grammes d'isoleucine par les 121,7 grammes de phénylalanine, pour obtenir la composition LAA2.

Exemple 8 préparation de N-undécylènoyl PhénylAlanine par la mise en œuvre du procédé 2 comparatif

On reproduit l'exemple 2 en remplaçant les 213,1 grammes d'acide palmitique par 40,5 grammes d'acide undécylénoyle, les 181,7 grammes d'isoleucine par les 50,4 grammes de phénylalanine, et les 100,7 grammes de N-palmitoyl isoleucine à pureté de 98% (pied de réaction) par 27,0 grammes de N-undécylènoyl PhénylAlanine à pureté de 98% (pied de réaction), pour obtenir la composition LAA21.

Les conditions opératoires des exemples 7 et 8, et les caractéristiques analytiques des compositions LAA2 et LAA21 correspondantes, sont consignées dans le tableau 2 ci-après.

[Table 2]

| Référence des compositions | LAA21 | LAA2 |
|---|---|---|
| **PROCEDE** | **2** | **1 selon l'invention** |
| **Ratio molaire Acide undécylènoique /Phényl Alanine** | 0,72 | 0,8 |
| **% massique de « pied de réaction »** | **30%** | **0%** |
| **Température de réaction** | **155°C** | **180°C** |
| **Durée du maintien en température de réaction** | 17h30 | 7h00 |
| **Analyses** | | |
| **Aspect à 25°C** | Solide marron | Solide Jaune |
| **Indice d'Alcalinité (méq/g)** | 0,274 | 0,104 |
| **Dosage acide undécylènoique résiduel (% massique)** | **7,5** | **5,3** |
| **Taux conversion molaire Acide undécylènoique (%)** | **79** | **90** |
| **PhénylAlanine en % aire** | 5,7 | n.d. |
| **Di-PhénylAlanine en % aire** | 6,9 | 4,4 |

Influence de la nature du procédé de préparation de compositions à base de N-Undécénoyl Phénylalanine.

Il apparaît clairement dans les exemples 7 et 8, que le procédé 1 selon l'invention permet d'obtenir une composition LAA2 avec une faible teneur en acide undécylènoyle (5,3%) et un fort taux de conversion de l'acide undécylènoyle (90%).

**III- Exemples de préparation de N-Palmitoyl Leucine par le procédé selon l'invention et par le procédé 3 comparatif**

Exemple 9 préparation de N-palmitoyl Leucine par la mise en œuvre du procédé 1 selon l'invention

On reproduit l'exemple 1 en remplaçant les 213,1 grammes d'acide palmitique par 244,1 grammes d'acide palmitique, les 181,7 grammes d'isoleucine par les 207,8 grammes de leucine, pour obtenir la composition LAA3.

Exemple 10 préparation de N-palmitoyl Leucine par la mise en œuvre du procédé 3 comparatif

On reproduit l'exemple 9 avec une température de maintien de la réaction de 150°C au lieu de 180°C, pour obtenir la composition LAA31.

Les conditions opératoires des exemples 9 et 10, et les caractéristiques analytiques des compositions LAA3 et LAA31 correspondantes, sont consignées dans le tableau 3 ci-après.

[Table 3]

| | C16 Leucine | |
|---|---|---|
| **Référence des compositions** | **LAA31** | **LAA3** |
| **Procédé** | **3** | **1 selon l'invention** |
| **ratio molaire Acide palmitique/Leucine** | 0,6 | 0,6 |
| **Température de réaction** | 150°C | 180°C |
| **Durée du maintien à la température de réaction** | 25h00 | 10h00 |
| **Analyses** | | |
| **Aspect à 25°C** | Solide marron | Solide Blanc rosé |
| **Indice d'Alcalinité (méq/g)** | 2,78 | 0,044 |
| **Dosage Acide Palmitique résiduel (% massique)** | **34,5** | **7,7** |
| **Taux conversion molaire Acide Palmitique (%)** | **38,5** | **85,8** |

(suite)

| Analyses | | |
|---|---|---|
| **Leucine résiduelle en % aire** | 40,0 | / |
| **% aire Acide Gras** | 35,1 | 18,4 |
| **Dl-Leucine % aire** | 7,0 | 3,5 |
| Influence de la nature du procédé de préparation de compositions à base de N-Palmitoyl Leucine. | | |

Il apparaît clairement dans les exemples 9 et 10, que le procédé 1 selon l'invention permet d'obtenir une composition LAA3 avec une faible teneur en acide palmitique (7,7%) et un fort taux de conversion de l'acide undécylènoyle (85,8%), et que la composition LAA21, obtenue selon le procédé 3, se caractérise par une fort teneur en acide palmitique résiduel (34,5%) et un faible taux de conversion de l'acide palmitique (38,5%).

**IV- Exemples de préparation de N-Palmitoyl PhénylAlanine par le procédé selon l'invention et par le procédé 3 comparatif**

Exemple 11 préparation de N-palmitoyl PhénylAlanine par la mise en œuvre du procédé 1 selon l'invention

On reproduit l'exemple 1 en remplaçant les 213,1 grammes d'acide palmitique par 142,0 grammes d'acide palmitique, les 181,7 grammes d'isoleucine par les 184,6 grammes de phénylalanine, pour obtenir la composition LAA4.

Exemple 12 préparation de N-palmitoyl PhénylAlanine par la mise en œuvre du procédé 3 comparatif

On reproduit l'exemple 11 avec une température de maintien de la réaction de 150°C au lieu de 180°C, pour obtenir la composition LAA41.

Les conditions opératoires des exemples 11 et 12, et les caractéristiques analytiques des compositions LAA4 et LAA41 correspondantes, sont consignées dans le tableau 4 ci-après.

[Table 4]

| **Références des compositions** | **LAA41** | **LAA4** |
|---|---|---|
| **Procédé** | **3** | **1 selon l'invention** |
| **ratio molaire Acide Palmitique / PhénylAlanine** | 0,7 | 0,5 |
| **Température de réaction** | 150°C | 180°C |
| **Durée du maintien à la température de réaction** | 19h45 | 6h20 |
| **Analyses** | | |
| **Aspect à 25°C** | Solide marron | Solide dur jaune |
| **Indice d'Alcalinité (milliéquivalent/g)** | 1,902 | 0,02 |
| **Dosage Acide Palmitique (% massique)** | **31,6** | **16,3** |
| **Taux conversion molaire Acide Palmitique (%)** | **41,4** | **64,8** |
| **Phényl Alanine (en % aire)** | 34,4 | / |
| **% aire Acide Gras** | 36,0 | 34,6 |
| **Di- Phényl Alanine (en % aire)** | 7,9 | 8,0 |
| Influence de la nature du procédé de préparation de compositions à base de Palmitoyl PhénylAlanine. | | |

Il apparaît clairement dans les exemples 11 et 12, que le procédé 1 selon l'invention permet d'obtenir une composition LAA4 avec une plus faible teneur en acide palmitique (16,3%) et un taux élevé de conversion de l'acide palmitique (64,8%), et que la composition LAA41, obtenue selon le procédé 3, se caractérise par une plus forte teneur en acide palmitique résiduel (31,6%) et un plus faible taux de conversion de l'acide palmitique (41,4%).

**V- Exemples de préparation de N-Lauroyl Isoleucine par le procédé selon l'invention et par le procédé 3 comparatif**

Exemple 13 préparation de N-lauroyl Isoleucine par la mise en œuvre du procédé 1 selon l'invention

On reproduit l'exemple 1 en remplaçant les 213,1 grammes d'acide palmitique par 140,0 grammes d'acide laurique, les 181,7 grammes d'isoleucine par les 152,0 grammes d'isoleucine, pour obtenir la composition LAA5.

Exemple 14 préparation de N-lauroyl Isoleucine par la mise en œuvre du procédé 3 comparatif

On reproduit l'exemple 13 avec une température de maintien de la réaction de 150 °C au lieu de 180°C, pour obtenir la composition LAA51.

Les conditions opératoires des exemples 13 et 14, et les caractéristiques analytiques des compositions LAA5 et LAA51 correspondantes, sont consignées dans le tableau 5 ci-après.

[Table 5]

| Référence des compositions | **LAA51** | **LAA5** |
|---|---|---|
| **Procédé** | **3** | **1 selon l'invention** |
| **Equivalent Acide gras/Isoleucine** | 0,6 | 0,6 |
| **Température de réaction** | 150°C | 180°C |
| **Durée du maintien de la température de réaction** | 15h00 | 4h30 |
| Analyses | | |
| **Aspect à 25°C** | Pâte brune | Pâte brune |
| **Indice d'Alcalinité (méq/g)** | 1,356 | 0,017 |
| **Dosage Acide laurique (%)** | **22,2** | **10,1** |
| **Taux conversion molaire d'acide laurique (%)** | **55,6** | **80,4** |
| **Isoleucine résiduelle (en % aire)** | 40,5 | / |
| **% aire Acide Gras** | 24,9 | 20,4 |
| **Di-Isoleucine (en % aire)** | 3,4 | 2,3 |
| Influence de la nature du procédé de préparation de compositions à base de N-Lauroyl Isoleucine | | |

Il apparaît clairement dans les exemples 13 et 14, que le procédé 1 selon l'invention permet d'obtenir une composition LAA5 avec une plus faible teneur en acide laurique (10,1%) et un taux élevé de conversion de l'acide laurique (80,4%), et que la composition LAA51, obtenue selon le procédé 3, se caractérise par une plus forte teneur en acide laurique résiduel (22,2%) et un plus faible taux de conversion de l'acide laurique (55,6%).

**VI- Exemple de préparation de dérivés N-octanoyl leucine par le procédé selon l'invention.**

Exemple 15 préparation de N-octanoyl leucine par la mise en œuvre du procédé 1 selon l'invention

On reproduit l'exemple 1 en remplaçant les 213,1 grammes d'acide palmitique par 214,7 grammes d'acide octanoïque, les 181,7 grammes d'isoleucine par les 244,5 grammes de leucine, pour obtenir la composition LAA6.

Les conditions opératoires de l'exemples 15, et les caractéristiques analytiques de la composition LAA6 correspondante, sont consignées dans le tableau 6 ci-après.

[Table 6]

| **Référence** | **LAA6** |
|---|---|
| **Procédé** | **1 selon l'invention** |
| **Ratio molaire Acide palmitique/Proline** | 0,8 |
| **Température de réaction** | 180°C |

(suite)

| Référence | LAA6 |
|---|---|
| **Durée du maintien de la température de réaction** | 4h45 |
| **Aspect à 25°C** | Pâte jaune |
| **Indice d'Alcalinité (méq/g)** | 0,026 |
| **Dosage Acide octanoique résiduel (%)** | **20,3** |
| **Taux conversion molaire Acide octanoïque (%)** | **59,8** |
| **Leucine résiduelle (en % aire)** | / |
| **Di-Leucine (en % aire)** | 11,9 |
| Composition et caractéristiques analytiques de la composition LAA6 obtenue par le procédé 1 selon l'invention. | |

L'exemple 15, par la mise en œuvre du procédé 1 selon l'invention, permet d'obtenir une composition LAA6 avec une faible teneur en acide octanoïque (20,1%) et un taux élevé de conversion de l'acide octanoïque (59,8%).

**VII- Exemples de préparation de dérivés N-acylé de la proline par le procédé selon l'invention.**

Exemple 16 préparation de N-palmitoyl Proline par la mise en œuvre du procédé 1 selon l'invention

On reproduit l'exemple 1 en remplaçant les 213,1 grammes d'acide palmitique par 95,8... grammes d'acide palmitique, les 181,7 grammes d'isoleucine par les 71,6 grammes de proline, et en introduisant progressivement la proline sur l'acide gras, pour obtenir la composition LAA7.

Exemple 17 préparation de N-stéaroyl Proline par la mise en œuvre du procédé 1 selon l'invention

On reproduit l'exemple 1 en remplaçant les 213,1 grammes d'acide palmitique par 79,2 grammes d'acide stéarique, les 181,7 grammes d'isoleucine par les 53,5 grammes de proline, et en introduisant progressivement la proline sur l'acide gras, pour obtenir la composition LAA8.

Exemple 18 préparation de N-Oléyl Proline par la mise en œuvre du procédé 1 selon l'invention

On reproduit l'exemple 1 en remplaçant les 213,1 grammes d'acide palmitique par 102,4 grammes d'acide oléique, les 181,7 grammes d'isoleucine par les 69,3 grammes de proline, et en introduisant progressivement la proline sur l'acide gras, pour obtenir la composition LAA9.

Les conditions opératoires des exemples 16, 17 et 18, et les caractéristiques analytiques des compositions LAA7, LAA8 et LAA9 correspondantes, sont consignées dans le tableau 7 ci-après.

[Table 7]

| Référence | LAA7 | LAA8 | LAA9 |
|---|---|---|---|
| **Procédé** | **1 selon l'invention** | **1 selon l'invention** | **1 selon l'invention** |
| **Ratio molaire Acide palmitique/Proline** | 0,6 | - | - |
| **Ratio molaire Acide stéarique/Proline** | - | 0,6 | - |
| **Ratio molaire Acide oléique/Proline** | - | - | 0,6 |
| **Température de réaction** | 180°C | 180°C | 180°C |
| **Durée d'introduction de la proline** | 1h45 | 1h30 | 1h15 |
| **Durée du maintien de la température de réaction** | 1h00 | 1h00 | 1h00 |
| **Aspect à 25°C** | Pâte jaune | Solide jaune | Liquide jaune |
| **Indice d'Alcalinité (méq/g)** | 0,015 | 0,011 | 0,015 |
| **Dosage Acide palmitique résiduel (%)** | **16,9** | - | - |

(suite)

| Référence | LAA7 | LAA8 | LAA9 |
|---|---|---|---|
| **Dosage Acide stéarique résiduel (%)** | - | **18,3** | - |
| **Dosage Acide oléique résiduel (%)** | - | - | **13,5** |
| **Taux conversion molaire Acide palmitique (%)** | **72,4** | - | - |
| **Taux conversion molaire Acide stéarique (%)** | - | **71,3** | - |
| **Taux conversion molaire Acide oléique (%)** | - | - | **78,8** |
| **Proline résiduelle (en % aire)** | / | - | - |
| **Di-Proline (en % aire)** | 13,7 | 12,9 | 13,0 |

Composition et caractéristiques analytiques des compositions LAA7, LAA8 et LAA9 obtenues par le procédé 1 selon l'invention.

Dans les exemples 16, 17 et 18, la mise en œuvre du procédé 1 selon l'invention, permet d'obtenir des compositions avec une faible teneur en acide gras résiduels (16,9% d'acide palmitique dans LAA7, 18,3% d'acide stéarique dans LAA8 et 13,5% d'acide oléique dans LAA9) et avec un fort taux de conversion d'acide gras (72,4% pour l'acide palmitique dans LAA7, 71,3% pour l'acide stéarique dans LAA8 et 78,8% pour l'acide oléique dans LAA9).

## Revendications

**1.** Procédé de préparation d'un N-acyle d'acide aminé comprenant la réaction d'un acide gras et d'un acide aminé à une température supérieure ou égale à 165°C pendant au moins 1h.

**2.** Procédé selon la revendication 1, **caractérisé en ce que** le N-acyle d'acide aminé est un dérivé N-acylé d'acides aminés de formule (I) :

R1-C(=O)-Y-OH (I),

dans laquelle R1-C(=O) représente un radical acyle, linéaire ou ramifié, saturé ou insaturé, comportant de 2 à 36 atomes de carbone et Y représente soit un radical de formule (Ia) :
N(R3)-CH(R2)-C(=O) (Ia), dans laquelle R3 représente l'atome d'hydrogène ou le radical méthyle, et R2 représente un atome d'hydrogène ou un radical alkyle, soit un radical de formule (Ib) :

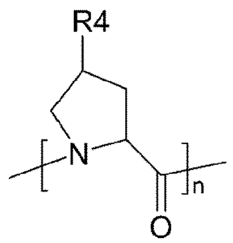

(Ib)

dans laquelle R4 représente l'atome d'hydrogène ou le radical hydroxyle.

**3.** Procédé selon l'une des revendications 1 ou 2, **caractérisé en ce que** l'acide gras est de formule (III) :

R1-C(=O)-OH (III),

dans laquelle R1-C(=O) représente un radical acyle, linéaire ou ramifié, saturé ou insaturé, comportant de 2 à 36 atomes de carbone
avec au moins un acide aminé de formule (IIa) :

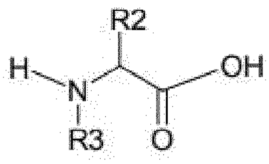

(IIa),

dans laquelle R3 représente l'atome d'hydrogène
ou le radical méthyle, et R2 représente un atome d'hydrogène ou un radical alkyle, ou de formule (IIb) :

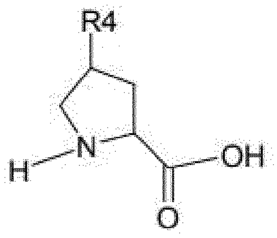

(IIb),

dans laquelle R4 représente un atome d'hydrogène ou un radical hydroxy.

**4.** Procédé selon l'une des revendications 2 ou 3, **caractérisé en ce que** le radical R2 représente un atome d'hydrogène ou un radical choisi parmi les radicaux méthyle, isopropyle, isobutyle, 1-méthyl propyle, benzyle, 3- amino propyle, hydroxyméthyle, 1-hydroxy éthyle, thiométhyle, (2-méthylthio) éthyle, 4-amino butyle, 3-guanidino propyle, 3-uréido propyle, (1-amino carbonyl) méthyle, carboxy méthyle, 2-carboxy éthyle, 2-(amino carbonyl) éthyle, 4-hydroxy benzyle, 3,4-dihydroxy benzyle, [1H-indol-3-yl] méthyle, (1H-imidazol-4-yl) méthyle.

**5.** Procédé selon l'une des revendications 1 à 4, **caractérisé en ce qu'**il comprend une étape d'introduction simultanée dans un réacteur de l'acide gras et de l'acide aminé.

**6.** Procédé selon l'une ou quelconque des revendications 1 à 5, **caractérisé en ce que** le ratio molaire entre l'acide aminé et l'acide gras est compris entre 0,5 et 1,0

**7.** Procédé selon l'une des revendications 1 à 6, **caractérisé en ce qu'**il est réalisé sous atmosphère d'azote.

**8.** Procédé selon l'une des revendications 1 à 7 comprenant :

- Une étape a) dans laquelle l'acide gras est porté à une température supérieure ou égale à 165°C,
- Une étape b) dans laquelle l'acide aminé sous forme solide est versé en une ou plusieurs fois, à une température supérieure ou égale à 150°C, sur l'acide gras issu de l'étape a) pour obtenir un mélange M1, et
- Une étape c) dans laquelle ledit mélange M1 est maintenu sous agitation mécanique pendant au moins une durée d'une heure et à une température supérieure ou égale à 165°C, pour obtenir un mélange aqueux M2 comprenant le N-acyle d'acide aminé.

**9.** Procédé selon la revendication 8, **caractérisé en ce que** pendant l'étape c) l'indice d'alcalinité du mélange M1 est mesurée jusqu'à atteindre une valeur inférieure ou égale à 0,2 milli équivalent/gramme de mélange M1.

**10.** Procédé selon l'une des revendications 1 à 7 comprenant :

- Une étape a) dans laquelle l'acide gras est porté à une température supérieure ou égale à 165°C,
- Une étape b) dans laquelle l'acide aminé sous forme solide est versé en une ou plusieurs fois, à une température comprise entre 80°C et 120°C, sur l'acide gras issu de l'étape a) pour obtenir un mélange M1,

- Une étape c) dans laquelle ledit mélange M1 est chauffé à une température supérieure ou égale à 165°C, et
- Une étape d) dans laquelle ledit mélange M1 est maintenu sous agitation mécanique pendant au moins une heure et à une température supérieure ou égale à 165°C pour obtenir un mélange aqueux M2 comprenant le N-acyle d'acide aminé.

**11.** Procédé selon la revendication 10, **caractérisé en ce que** pendant l'étape d) l'indice d'alcalinité du mélange M1 est mesuré jusqu'à atteindre une valeur inférieure ou égale à 0,2 milliéquivalent/gramme de mélange M1.

**12.** Procédé selon l'une des revendications 8 à 11, **caractérisé en ce que** l'étape a) comprend une première sous-étape de fonte de l'acide gras et une deuxième sous-étape d'homogénéisation de l'acide gras.

**13.** Procédé selon l'une des revendications 8 à 12, **caractérisé en ce qu'**il comprend une étape supplémentaire d'isolation du N-acyle d'acide aminé du mélange M2.

**14.** Procédé de fabrication d'une composition cosmétique comprenant la préparation d'un N-acyle d'acide aminé telle que défini à l'une des revendications 1 à 13.

**15.** Procédé suivant la revendication 14, comprenant la préparation d'un N-acyle d'acide aminé telle que défini à l'une des revendications 8 à 12 et dans lequel la composition cosmétique est une composition cosmétique à usage topique (C) comprenant pour 100% de sa masse :

- de 0,1% à 40% massique du mélange (M2), et
- de 60% à 99,9% massique d'un milieu cosmétiquement acceptable.

## Patentansprüche

**1.** Verfahren zur Herstellung einer n-Acylaminosäure, das die Umsetzung einer Fettsäure mit einer Aminosäure bei einer Temperatur von mehr als oder gleich 165 °C für mindestens 1 h umfasst.

**2.** Verfahren nach Anspruch 1, **dadurch gekennzeichnet, dass** es sich bei der n-Acylaminosäure um ein n-Acylaminosäure-Derivat der Formel (I):

R1-C(=O)-Y-OH (I)

handelt, wobei R1-C(=O) für einen gesättigten oder ungesättigten, geradkettigen oder verzweigten, 2 bis 36 Kohlenstoffatome aufweisenden Acylrest steht und Y entweder für einen Rest der Formel (Ia):
N(R3)-CH(R2)-C(=O) (Ia), wobei R3 für ein Wasserstoffatom oder einen Methylrest steht und R2 für ein Wasserstoffatom oder einen Alkylrest steht, oder für einen Rest der Formel (Ib):

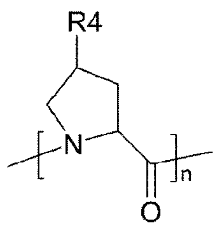

(Ib)

steht, wobei R4 für ein Wasserstoffatom oder einen Hydroxylrest steht.

**3.** Verfahren nach einem der Ansprüche 1 oder 2, **dadurch gekennzeichnet, dass** die Fettsäure die Formel (III):

R1-C(=O)-OH (III)

aufweist, wobei R1-C(=O) für einen gesättigten oder ungesättigten, geradkettigen oder verzweigten, 2 bis 36 Kohlenstoffatome aufweisenden Acylrest steht,
mit mindestens einer Aminosäure der Formel (IIa):

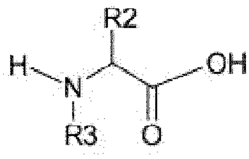

(IIa),

wobei R3 für ein Wasserstoffatom oder einen Methylrest steht und R2 für ein Wasserstoffatom oder einen Alkylrest steht,
oder der Formel (IIb):

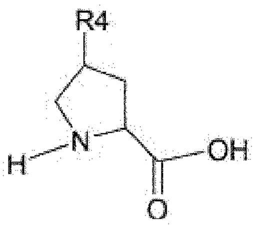

(IIb),

, wobei R4 für ein Wasserstoffatom oder einen Hydroxyrest steht.

**4.** Verfahren nach einem der Ansprüche 2 oder 3, **dadurch gekennzeichnet, dass** der Rest R2 für ein Wasserstoffatom oder einen Rest steht, der aus einem Methyl-, Isopropyl, Isobutyl-, 1-Methylpropyl-, Benzyl-, 3-Aminopropyl-, Hydroxymethyl-, 1-Hydroxyethyl-, Thiomethyl-, (2-Methylthio)ethyl-, 4-Aminobutyl-, 3-Guanidinopropyl-, 3-Ureidopropyl-, (1-Aminocarbonyl)methyl-, Carboxymethyl-, 2-Carboxyethyl-, 2-(Aminocarbonyl)ethyl-, 4-Hydroxybenzyl-, 3,4-Dihydroxybenzyl-, [1H-Indol-3-yl]methyl-, (1H-Imidazol-4-yl)methyl-Rest ausgewählt ist.

**5.** Verfahren nach einem der Ansprüche 1 bis 4, **dadurch gekennzeichnet, dass** es einen Schritt des gleichzeitigen Einleitens der Fettsäure und der Aminosäure in einen Reaktor umfasst.

**6.** Verfahren nach einem oder mehreren der Ansprüche 1 bis 5, **dadurch gekennzeichnet, dass** das Molverhältnis zwischen der Aminosäure und der Fettsäure zwischen 0,5 und 1,0 liegt.

**7.** Verfahren nach einem der Ansprüche 1 bis 6, **dadurch gekennzeichnet, dass** es unter einer Stickstoffatmosphäre erfolgt.

**8.** Verfahren nach einem der Ansprüche 1 bis 7, dass Folgendes umfasst:

- einen Schritt a), in dem die Fettsäure auf eine Temperatur von mehr als oder gleich 165 °C gebracht wird,
- einen Schritt b), in dem die Aminosäure in fester Form in einer oder mehreren Portionen bei einer Temperatur von mehr als oder gleich 150 °C zu der Fettsäure von Schritt a) gegeben wird, wodurch eine Mischung M1 erhalten wird, und
- einen Schritt c), in dem die Mischung M1 unter mechanischem Rühren zumindest für eine Dauer von einer Stunde auf einer Temperatur von mehr als oder gleich 165 °C gehalten wird, wodurch eine wässrige Mischung M2 erhalten wird, welche die n-Acylaminosäure umfasst.

**9.** Verfahren nach Anspruch 8, **dadurch gekennzeichnet, dass** in Schritt c) die Basenzahl der Mischung M1 gemessen wird, bis ein Wert von kleiner als oder gleich 0,2 Milliäquivalent/Gramm von Mischung M1 erreicht ist.

**10.** Verfahren nach einem der Ansprüche 1 bis 7, dass Folgendes umfasst:

- einen Schritt a), in dem die Fettsäure auf eine Temperatur von mehr als oder gleich 165 °C gebracht wird,

- einen Schritt b), in dem die Aminosäure in fester Form in einer oder mehreren Portionen bei einer Temperatur zwischen 80 °C und 120 °C zu der Fettsäure von Schritt a) gegeben wird, wodurch eine Mischung M1 erhalten wird,
- einen Schritt c), in dem die Mischung M1 auf eine Temperatur von mehr als oder gleich 165 °C erwärmt wird, und
- einen Schritt d), in dem die Mischung M1 unter mechanischem Rühren für mindestens eine Stunde auf einer Temperatur von mehr als oder gleich 165 °C gehalten wird, wodurch eine wässrige Mischung M2 erhalten wird, welche die n-Acylaminosäure umfasst.

**11.** Verfahren nach Anspruch 10, **dadurch gekennzeichnet, dass** in Schritt d) die Basenzahl der Mischung M1 gemessen wird, bis ein Wert von kleiner als oder gleich 0,2 Milliäquivalent/Gramm von Mischung M1 erreicht ist.

**12.** Verfahren nach einem der Ansprüche 8 bis 11, **dadurch gekennzeichnet, dass** Schritt a) einen ersten Teilschritt eines Schmelzens der Fettsäure und einen zweiten Teilschritt eines Homogenisierens der Fettsäure umfasst.

**13.** Verfahren nach einem der Ansprüche 8 bis 12, **dadurch gekennzeichnet, dass** es einen zusätzlichen Schritt eines Isolierens der n-Acylaminosäure aus der Mischung M2 umfasst.

**14.** Verfahren zur Herstellung einer kosmetischen Zusammensetzung, das die Herstellung einer n-Acylaminosäure gemäß der Definition in einem der Ansprüche 1 bis 13 umfasst.

**15.** Verfahren nach Anspruch 14, das die Herstellung einer n-Acylaminosäure gemäß der Definition in einem der Ansprüche 8 bis 12 umfasst und wobei es sich bei der kosmetischen Zusammensetzung um eine kosmetische Zusammensetzung (C) zur topischen Verwendung handelt, die auf 100 % ihrer Masse Folgendes umfasst:

- 0,1 Gew.-% bis 40 Gew.-% Mischung (M2) und
- 60 Gew.-% bis 99,9 Gew.-% eines kosmetisch annehmbaren Mediums.

## Claims

**1.** Method for preparing an N-acylamino acid, comprising the reaction of a fatty acid and an amino acid at a temperature greater than or equal to 165°C for at least 1 h.

**2.** Method according to Claim 1, **characterized in that** the N-acylamino acid is an N-acyl derivative of amino acids of formula (I):

R1-C(=O) -Y-OH (I),

in which R1-C(=O) represents a linear or branched, saturated or unsaturated acyl radical containing from 2 to 36 carbon atoms and Y represents either a radical of formula (Ia):
N(R3)-CH(R2)-C(=O) (Ia), in which R3 represents the hydrogen atom or the methyl radical and R2 represents a hydrogen atom or an alkyl radical, i.e. a radical of formula (Ib):

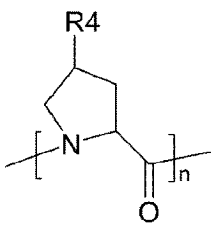

(Ib),

in which R4 represents the hydrogen atom or the hydroxyl radical.

**3.** Method according to one of Claims 1 and 2, **characterized in that** the fatty acid is of formula (III):

R1-C(=O)-OH (III),

in which R1-C(=O) represents a linear or branched, saturated or unsaturated acyl radical containing from 2 to 36 carbon atoms
with at least one amino acid of formula (IIa):

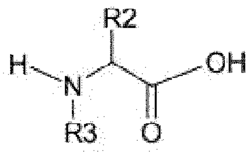

(IIa),

in which R3 represents the hydrogen atom or the methyl radical and R2 represents a hydrogen atom or an alkyl radical,
or of formula (IIb):

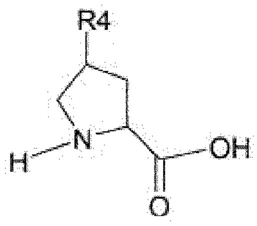

(IIb),

in which R4 represents a hydrogen atom or a hydroxyl radical.

**4.** Method according to one of Claims 2 and 3, **characterized in that** radical R2 represents a hydrogen atom or a radical selected from methyl, isopropyl, isobutyl, 1-methylpropyl, benzyl, 3-aminopropyl, hydroxymethyl, 1-hydroxyethyl, thiomethyl, (2-methylthio)ethyl, 4-aminobutyl, 3-guanidinopropyl, 3-ureidopropyl, (1-aminocarbonyl)methyl, carboxymethyl, 2-carboxyethyl, 2-(aminocarbonyl)ethyl, 4-hydroxybenzyl, 3,4-dihydroxybenzyl, [1H-indol-3-yl]methyl and (1H-imidazol-4-yl)methyl.

**5.** Method according to one of Claims 1 to 4, **characterized in that** it comprises a step of simultaneously introducing into a reactor the fatty acid and the amino acid.

**6.** Method according to any one of Claims 1 to 5, **characterized in that** the molar ratio between the amino acid and the fatty acid is between 0.5 and 1.0.

**7.** Method according to one of Claims 1 to 6, **characterized in that** it is performed under a nitrogen atmosphere.

**8.** Method according to one of Claims 1 to 7, comprising:

- a step a) in which the fatty acid is brought to a temperature greater than or equal to 165°C,
- a step b) in which the amino acid in solid form is poured in one or more portions, at a temperature greater than or equal to 150°C, onto the fatty acid obtained from step a) so as to obtain a mixture M1, and
- a step c) in which said mixture M1 is maintained under mechanical stirring for at least a period of one hour and at a temperature greater than or equal to 165°C so as to obtain an aqueous mixture M2 comprising the **N-**acylamino acid.

**9.** Method according to Claim 8, **characterized in that**, during step c), the base number of mixture M1 is measured until a value of less than or equal to 0.2 milliequivalents/gram of mixture M1 is attained.

**10.** Method according to one of Claims 1 to 7, comprising:

- a step a) in which the fatty acid is brought to a temperature greater than or equal to 165°C,
- a step b) in which the amino acid in solid form is poured in one or more portions, at a temperature of between 80°C and 120°C, onto the fatty acid obtained from step a) so as to obtain a mixture M1,
- a step c) in which said mixture M1 is heated to a temperature greater than or equal to 165°C, and
- a step d) in which said mixture M1 is maintained under mechanical stirring for at least one hour and at a temperature greater than or equal to 165°C so as to obtain an aqueous mixture M2 comprising the N-acylamino acid.

**11.** Method according to Claim 10, **characterized in that**, during step d), the base number of mixture M1 is measured until a value of less than or equal to 0.2 milliequivalents/gram of mixture M1 is attained.

**12.** Method according to one of Claims 8 to 11, **characterized in that** step a) comprises a first substep of melting the fatty acid and a second substep of homogenizing the fatty acid.

**13.** Method according to one of Claims 8 to 12, **characterized in that** it comprises an additional step of isolating the N-acylamino acid from mixture M2.

**14.** Method for producing a cosmetic composition comprising the preparation of an N-acylamino acid as defined in one of Claims 1 to 13.

**15.** Method according to Claim 14, comprising the preparation of an N-acylamino acid as defined in one of Claims 8 to 12 and in which the cosmetic composition is a cosmetic composition for topical use (C) comprising, per 100% of its weight:

- from 0.1% to 40% by weight of the mixture (M2) and
- from 60% to 99.9% by weight of a cosmetically acceptable medium.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2463779 A
- US 6703517 B
- WO 9221318 A
- WO 9426694 A
- FR 3066195 A
- FR 3066194 A
- US 20160052869 A
- FR 3066194


### Littérature non-brevet citée dans la description


- *J. Am. Oil Chem. Soc.*, 1956, vol. 78, 172